# EUROPEAN PATENT APPLICATION

(11) **EP 2 055 223 A1**
(43) Date of publication of application: **06.05.2009**
(21) Application number: 07767011.5
(22) Date of filing: 31.05.2007
(51) Int. Cl.: A61B 1/06, G02B 23/26

(54) **ENDOSCOPE DEVICE**

(30) Priority: 24.08.2006 JP 2006228191
(71) Applicant: Olympus Medical Systems Corp., Tokyo 151-0072 (JP)
(72) Inventor: NIIDA, Koichi, Tokyo, 151-0072 (JP)
(74) Representative: von Hellfeld, Axel
(86) International application number: PCT/JP2007/061089
(87) International publication number: WO 2008/023488

(57) **Abstract**

An endoscope apparatus 1 of the present invention includes: a CCD 12; a plurality of LEDs 11a to 11d for illuminating the field of vision for image pickup of the CCD 12; and a dimming circuit 9 for detecting the brightness in the illumination area of each of the plurality of LEDs 11a to 11d using the video signal obtained by the CCD 12 and dimming light for each of the LEDs 11a to 11d based on the detected result, and the LEDs 11a to 11d are individually provided with respect to the CCD 12 at the positions corresponding to the illumination area of each of the LEDs 11a to 11d (the divided screens 16a to 16d) when the area dimming section 17 of the dimming circuit 9 detects the brightness of the illumination areas of the plurality of LEDs 11a to 11d.

## Description

### Technical Field

The present invention relates to an endoscope apparatus which includes an endoscope insertion portion having illumination means incorporated at the distal end portion thereof to be inserted into a body cavity.

### Background Art

Generally, a conventional endoscope apparatus is often provided with a light source apparatus separate from the endoscope. The endoscope is further provided with a light guide such as optical fiber therein. Thus, the light guide is connected to the light source apparatus at the proximal end portion thereof, so that an illumination light from the light source apparatus is introduced into the distal end portion of the endoscope insertion portion through the light guide to irradiate the outside of the endoscope from the distal end portion of the light guide, which illuminates a field of vision in the direction of observation by the endoscope.

Such an endoscope apparatus has a long light path for introducing an illumination light from the light source apparatus to the distal end portion of the light guide, which generates a quantity of light loss in a midway of the light path by which the illumination light from the light source apparatus is introduced to the distal end portion of the light guide and also requires a prevention of heat radiation that occurs in the light source apparatus.

Then, recently, a number of propositions for an endoscope apparatus have been made in view of the above situation, the endoscope apparatus having an endoscope insertion portion that includes illumination means such as an LED at the distal end portion thereof for preventing any loss of illumination light and reducing the steps for preventing heat radiation in the light source apparatus.

For example, Japanese Patent Application Laid-Open Publication No. 11-225952 discloses a technology for an endoscope apparatus which includes an endoscope insertion portion having a distal end portion with an observation window, nine LEDs provided near the window, and an automatic dimming circuit controlling the light quantity of the nine LEDs, so that the automatic dimming circuit detects the brightness of each luminance signal from a monitor screen which is divided into the same number of smaller screens as the nine LEDs, and controls an LED drive circuit so as to individually control the light quantity of each of the nine LEDs based on the detected result.

Also, for example, Japanese Patent Application Laid-Open Publication No. 2005-288191 discloses a technology for a capsule type in-vivo image picking up device which is provided with light sources such as a plurality of LEDs, and a light detecting device around the observation window at the distal end portion of the endoscope insertion portion, so that, based on the light quantity detected by the light detecting device, the light quantity of the plurality of light sources, light irradiation time, and the like can be controlled.

However, as described above, in the endoscope apparatus having illumination means such as an LED incorporated in the distal end portion thereof, the power supply voltage values supplied to all of the LEDs are simultaneously converted to control the light quantities of the illumination lights irradiated from the LEDs so that the light quantities can be controlled for the entire plurality of LEDs. As a result, the light quantities for the entire field of vision for observation by the endoscope can only be controlled simultaneously, resulting in that sometimes an adequate dimming cannot be obtained when a field of vision for observation by the endoscope having brighter parts and darker parts locally therein provides an observation image of the endoscope.

In addition, in the endoscope apparatus disclosed in Japanese Patent Application Laid-Open Publication No. 11-225952, the nine LEDs are arranged on one side of image pickup means at a distal end surface of the endoscope apparatus, thereby the light quantities are not well balanced with respect to an object, and also each part of the monitor screen which is divided into the same number of parts as the nine LEDs is not always arranged corresponding to the position of the object area the image of which is to be picked up by the image pickup means. As a result, sometimes a proper dimming cannot be achieved when a field of vision for observation by the endoscope having brighter parts and darker parts therein provides an observation image of the endoscope.

Furthermore, the in vivo device for pickup image of living body disclosed in Japanese Patent Application Laid-Open Publication No. 2005-288191 is configured with the light detecting devices which are arranged near and around the observation window, but the positions of the detected light quantities by the light detecting devices with respect to an object are not always corresponding to the position of the object area the image of which is to be picked up by the image pickup means as in case of the above endoscope apparatus, and the above described phenomenon has not been eliminated yet. Also, in the above patent document, there is no description about a dimming method and dimming means for dimming control by controlling light quantities of a plurality of light sources based on the light quantities detected by the light detecting apparatus, so that an entire monitor screen has a brightness that allows an operator to easily observe a picked image: thereby there is a need for dimming control in which light quantities of a plurality of light sources are controlled so as to provide a monitor screen having a brightness that allows an operator to easily observe the entire screen.

The present invention has been made in view of the above situation, and an object of the present invention is to provide an endoscope apparatus in which a proper dimming is performed for each illumination means and the dimming provides a monitor screen having a brightness that allows the entire screen to be easily observed even when an observation image of the endoscope has brighter parts and darker parts locally in a part of the entire field of vision for observation by the endoscope.

### Disclosure of the Invention

### Means for Solving the Problem

An endoscope apparatus of the present invention includes: a plurality of illumination means for illuminating a field of vision the image of which is picked up by the image pickup means; a dimming control section for detecting the brightness of the area illuminated by each of the plurality of illumination means using the video signals obtained from the image pickup means, and dimming the light of each of the illumination means based on the detected result, with the plurality of illumination means being arranged at the positions corresponding to the areas illuminated by each of the illumination means with respect to the image pickup means when the dimming control section detects the brightness of the areas illuminated by each of the plurality of illumination means.

### Brief Description of the Drawings

Fig. 1 is a configuration diagram schematically showing the entire system of an endoscope apparatus according to an embodiment 1 of the present invention;
Fig. 2 is a configuration diagram schematically showing a distal end portion of an endoscope insertion portion of Fig. 1;
Fig. 3 is a perspective diagram showing an observation window and the configuration of a plurality of LEDs at a distal end surface of the distal end portion of Fig. 2;
Fig. 4 is a configuration showing the divided areas of a monitor screen corresponding to the arrangement of image pickup means and the plurality of LEDs of Fig. 3;
Fig. 5 is a block diagram showing a specific configuration of a dimming circuit of Fig. 1;
Fig. 6 is a block diagram showing a specific configuration of an area dimming section of Fig. 5;
Fig. 7 is a block diagram showing a specific configuration of a screen brightness control section of Fig. 5;
Fig. 8 is a configuration diagram showing an electrical circuit configuration of the entire endoscope apparatus shown in Fig. 1;
Fig. 9A is a diagram illustrating various dimming signals to show the operation of the embodiment 1;
Fig. 9B is a diagram illustrating the processes for the various dimming signals to show the operation of the embodiment 1 together with Fig. 9A;
Fig. 10 is a diagram illustrating dimming in the prior art;
Fig. 11 is a diagram showing a display example of a monitor screen displayed by dimmed light shown in Fig. 10;
Fig. 12 is a diagram illustrating the dimming in the embodiment 1;
Fig. 13 is a display example of a monitor screen displayed by the dimmed light shown in Fig. 11;
Fig. 14 is a configuration diagram showing an electrical circuit configuration of an entire endoscope apparatus according to an embodiment 2 of the present invention;
Fig. 15 is an exploded perspective diagram showing the configuration of a connection mechanism used at the connection A of the endoscope 2 of Fig. 1;
Fig. 16 is a cross sectional diagram showing a fixed connection mechanism;
Fig. 17 is a perspective diagram showing the configuration of a connection mechanism at the connection B between a proximal end portion of the insertion portion and a hand-side end portion of the endoscope; and
Fig. 18 is a cross sectional diagram showing a distal end portion of the insertion portion which constitutes a rotary self-propelled endoscope and has a leading portion in front of the distal end portion thereof.

### Best mode for Carrying Out the Invention

Now, with reference to the drawings, embodiments of the present invention will be explained below.

### (Embodiment 1)

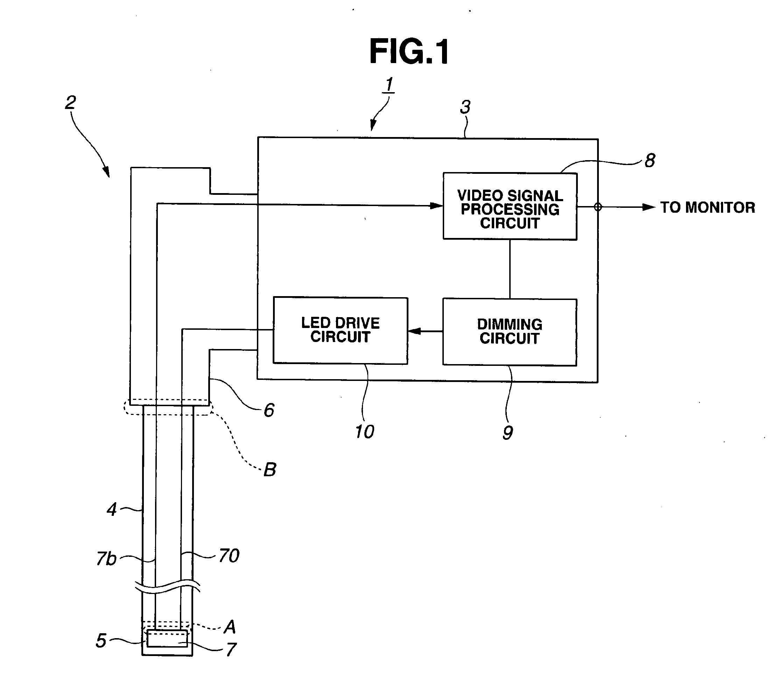

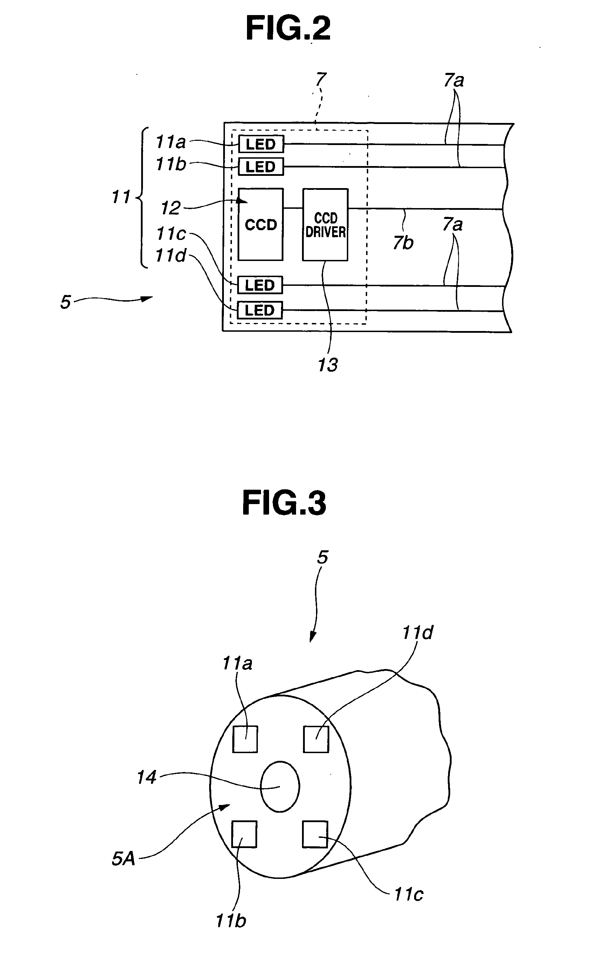

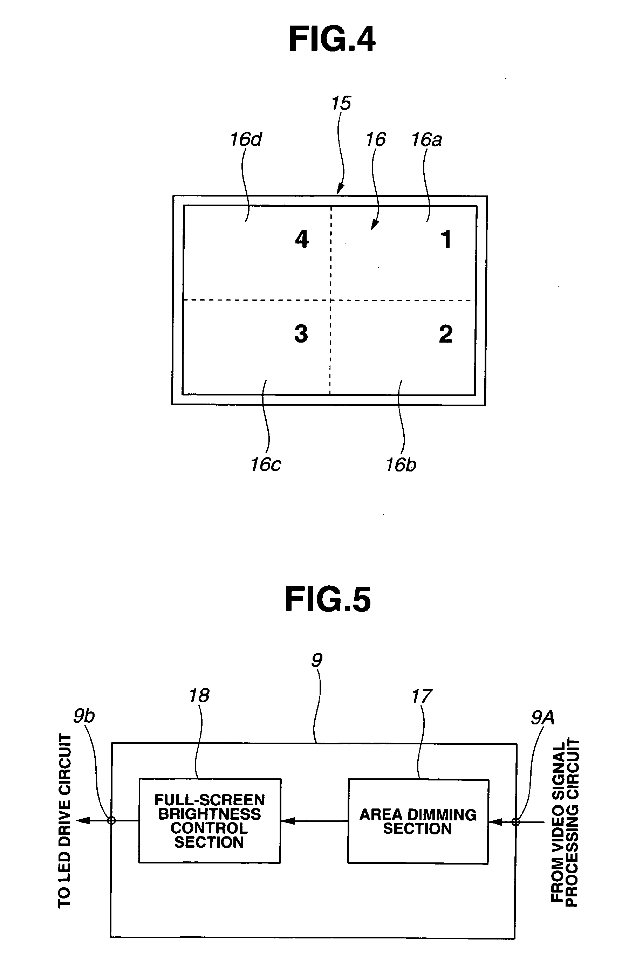

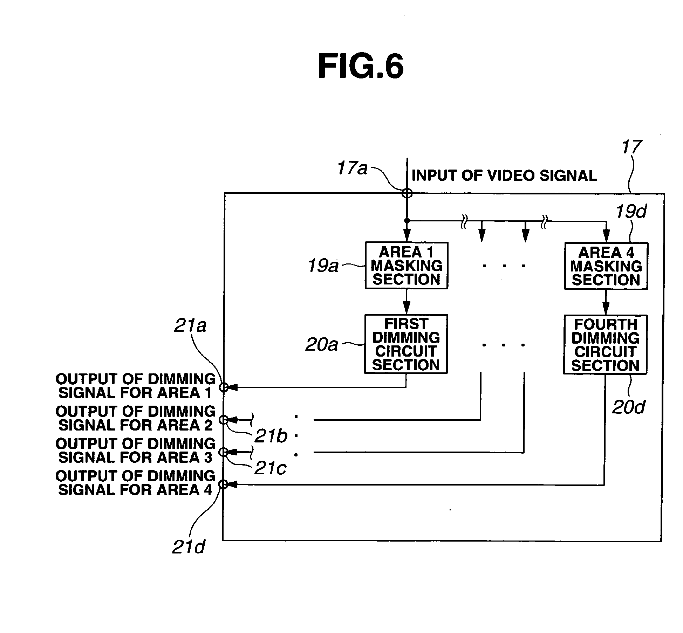

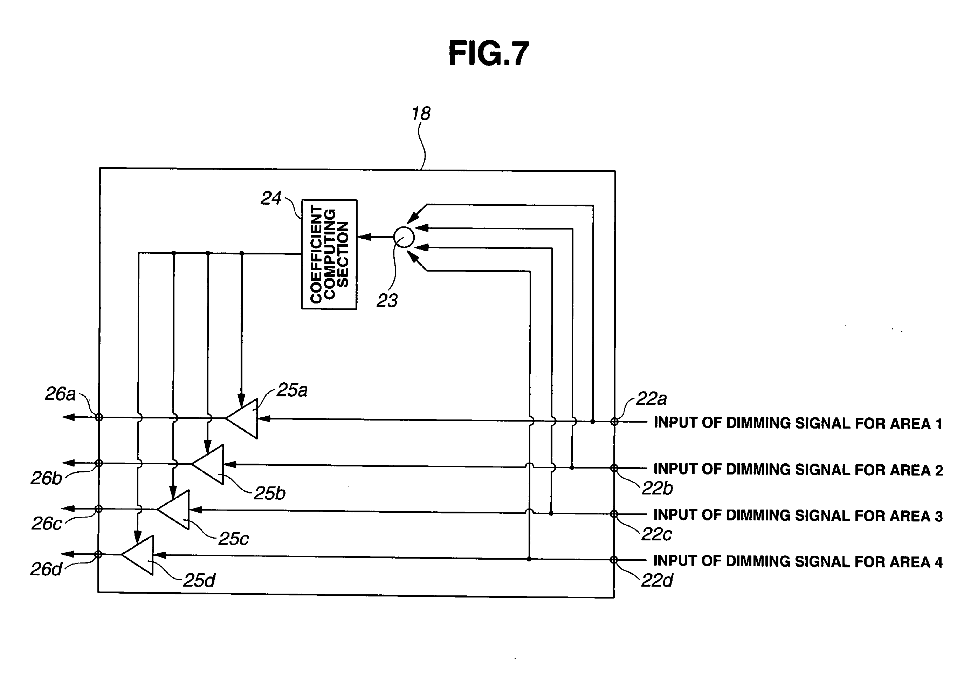

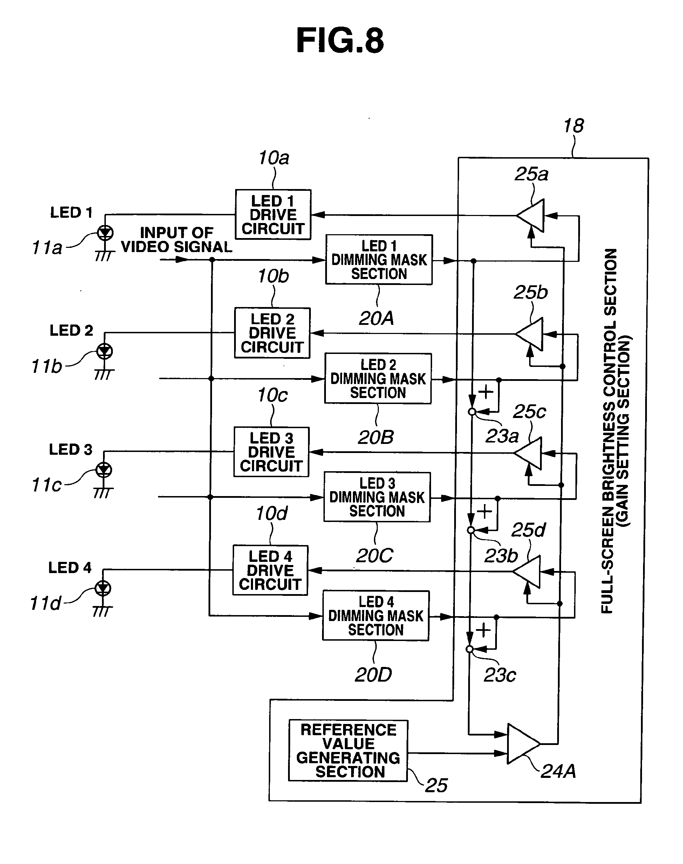

Figs. 1 to 13 show an embodiment 1 of the present invention. Fig. 1 is a configuration diagram schematically showing the entire system of an endoscope apparatus according to the embodiment 1. Fig. 2 is a configuration diagram schematically showing a distal end portion of an endoscope insertion portion of Fig. 1. Fig. 3 is a perspective diagram showing an observation window and the configuration of a plurality of LEDs at a distal end surface of the distal end portion of Fig. 2. Fig. 4 is a configuration showing the divided areas of a monitor screen corresponding to the arrangement of image pickup means and the plurality of LEDs of Fig. 3. Fig. 5 is a block diagram showing a specific configuration of a dimming circuit of Fig. 1. Fig. 6 is a block diagram showing a specific configuration of an area dimming section of Fig. 5. Fig. 7 is a block diagram showing a specific configuration of a screen brightness control section of Fig. 5. Fig. 8 is a configuration diagram showing an electrical circuit configuration of the entire endoscope apparatus shown in Fig. 1.

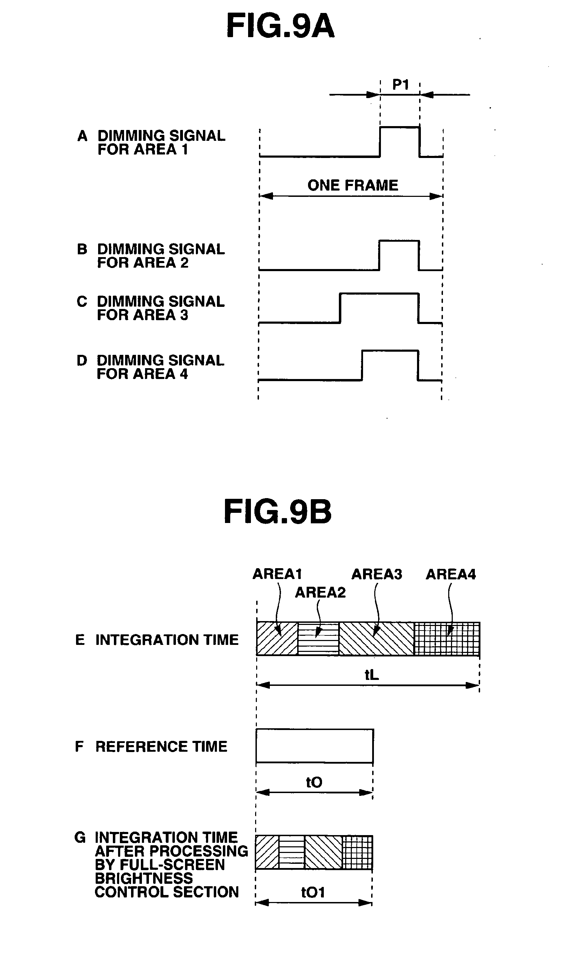

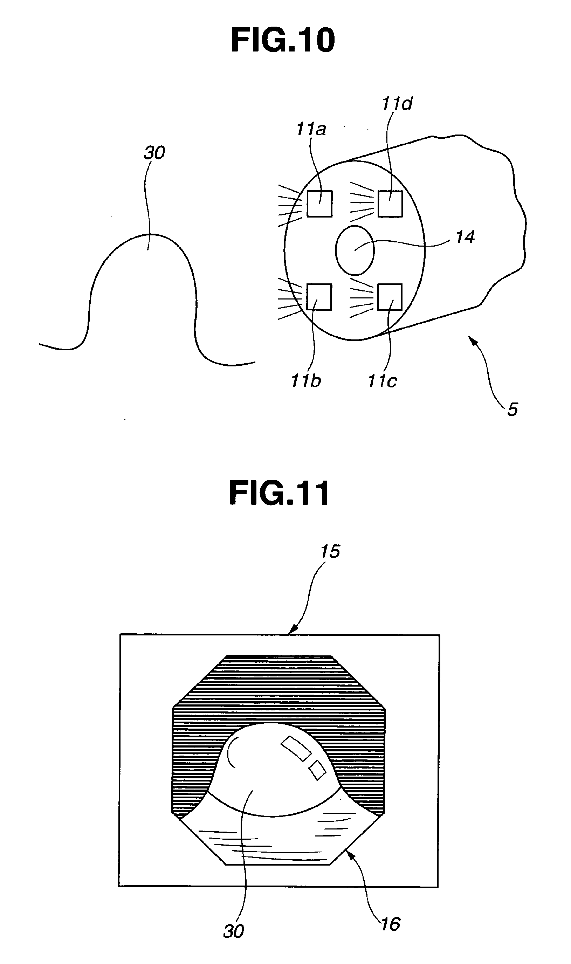

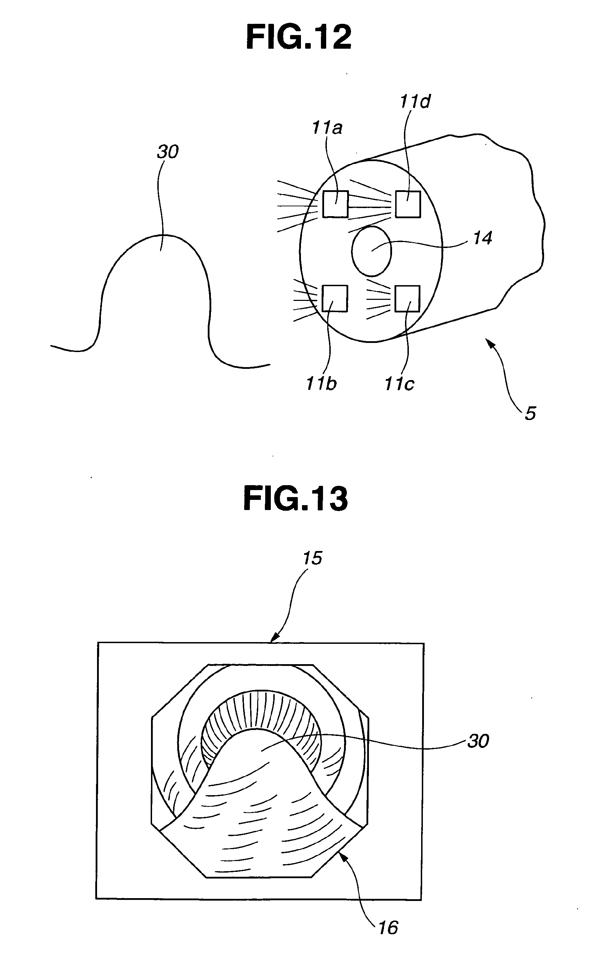

Also, Figs. 9A to 13 show the operation of the embodiment 1. Fig. 9A is a diagram illustrating various dimming signals. Fig. 9B is a diagram illustrating the processes for the various dimming signals. Fig. 10 is a diagram illustrating dimming in the prior art. Fig. 11 is a diagram showing a display example of a monitor screen displayed by dimmed light shown in Fig. 10. Fig. 12 is a diagram illustrating the dimming in the embodiment 1. Fig. 13 is a display example of a monitor screen displayed by the dimmed light shown in Fig. 11.

As shown in Fig. 1, an endoscope apparatus 1 of the embodiment 1 includes an endoscope 2 and a CCU (camera control unit) 3 which is a video processor.

The endoscope 2 is provided with an insertion portion 4 which is to be inserted into body cavity, and a hand-side end portion 6 connected to a proximal end portion of the insertion portion 4. The insertion portion 4 further includes a distal end portion 5 in which an image pickup unit 7 with illumination means 11 and image pickup means 12 is provided. The hand-side end portion 6 or the CCU 3 is configured as an operation section.

Here, the configurations of the image pickup unit 7 and the distal end portion 5 will be explained below.

The image pickup unit 7 includes, as shown in Fig. 2, a plurality of LEDs (white LEDs, semiconductor light emitting device), for example four LEDs 11a to 11d, which are the illumination means 11 for emitting illumination light, a CCD (solid state image pickup device) 12 which is means for picking up image for observation 12, and a CCD driver 13 for driving the CCD 12. The CCD driver 13 may be configured to be included in the CCD 3 which will be explained later.

The four LEDs 11a to 11d are individually electrically connected to an LED drive circuit 10 shown in Fig. 1 via connecting lines 7a. In front of the CCD 12 is provided an objective optical system (not shown), and an observation window 14 is arranged at a distal end surface 5A of the distal end portion 5 to cover the objective optical system (see Fig. 3). The CCD driver 13 is electrically connected to a video signal processing circuit 8 shown in Fig. 1 via a connecting line 7b.

In the present embodiment, the four LEDs 11a to 11d are, as shown in Fig. 3, arranged around the observation window 14 which is disposed in the front surface of the CCD 12, for example at the four positions corresponding to the four corners of a rectangular which is formed in the distal end surface 5A with the observation window 14 being at the center. In the case, the positional relationship between the four LEDs 11a to 11d and the CCD 12 corresponds to brightness detection areas for detecting each of the brightness of four illumination areas which will be explained later.

That is, an observation image picked up by the CCD 12, that is, a screen 16 of a monitor 15 shown in Fig. 4 is divided into four screens, the same number as the four LEDs 11a to 11d, and the brightness is detected from the luminance signal of each of the screens 16a to 16d which are subjected to a masking process using the video signal so as to correspond to the divided screens 16a to 16d respectively. Therefore, the screen 16a to 16d are the brightness detection areas corresponding to the four LEDs 11a to 11d, respectively.

In the case, for example, as shown in Figs. 3 and 4, the illumination area of the first LED 11a (the upper left part of the distal end surface 5A) corresponds to the first screen 16a of the monitor screen 16 (the upper right part of the monitor screen 16), while the illumination area of the second LED 11b (the lower left part of the distal end surface 5A) corresponds to the second screen 16b of the monitor screen 16 (the lower right part of the monitor screen 16). Similarly, the illumination area of the third LED 11c (the lower right part of the distal end surface 5A) corresponds to the third screen 16c of the monitor screen 16 (the lower left part of the monitor screen 16), while the illumination area of the fourth LED 11d (the upper right part of the distal end surface 5A) corresponds to the fourth screen 16d of the monitor screen 16 (the upper left part of the monitor screen 16).

In the present embodiment, as the illumination means 11, the four LEDs 11a to 11d are provided, but the illumination means 11 is not limited to those, and may be four or more LEDs 11. However, the provided four or more LEDs and the CCD 12 should be arranged, as described above, to individually correspond to each of the screen 16a to 16n of the same number of illumination areas (detection areas) as the four or more plurality of LEDs.

As shown in Fig. 1, the hand-side end portion 6 of the endoscope 2 is removably coupled to a scope coupling section of the CCU 3.

The CCU 3 includes: a video signal processing circuit 8 which is electrically connected to each of the CCD driver 13 and the output side of the CCD 12 in the image pickup unit 7 via the connecting line 7b; a dimming circuit 9 as a dimming control section to which a video signal is supplied from the video signal processing circuit 8 and sets a light quantity value of the illumination means 11 based on the video signal; and an LED drive circuit 10 which is electrically connected to each of the LEDs 11a to 11d in the image pickup unit 7, and drives each of the LEDs 11a to 11d based on the dimming signal from the dimming circuit 9.

The connecting line 7b is, not shown but, configured with a connecting line on the input side for supplying drive signals from the video signal processing circuit 8 to the CCD driver 13, and a connecting line on the output side for outputting the outputted signal from the CCD 12 to the video signal processing circuit 8.

The video signal processing circuit 8 is electrically connected with the monitor 15 shown in Fig. 4 via an output terminal 3a. In use of the endoscope 2, the CCD driver 13 drives the CCD 12. In the driving, an observation image of the endoscope 2 is converted to an electrical signal by the CCD 12 to be outputted. Then, the outputted signal from the CCD 12 is inputted into the video signal processing circuit 8 to be converted to a video signal, so that the video signal outputted from the video signal processing circuit 8 is inputted to the monitor 15 via the output terminal 3a, and then the observation image of the endoscope 2 is displayed on the screen 16 of the monitor 15.

The dimming circuit 9 is electrically connected with video signal processing circuit 8 and the LED drive circuit 10 individually. A part of the video signals outputted from the video signal processing circuit 8 is inputted to the dimming circuit 9, where the light quantity value of the illumination means 11 is set based on the video signal outputted from the video signal processing circuit 8, so that various dimming signals based on the set light quantity value are outputted to the LED drive circuit 10.

The LED drive circuit 10 includes a constant current pulse width modulation circuit (not shown) for controlling the illumination duration of the illumination means 11 for the four LEDs 11a to 11d, and also has individual light quantity control means incorporated therein for individually controlling the light quantity of the LEDs 11a to 11d. Thus, the LED drive circuit 10 is configured to individually control the light quantity of the four LEDs 11a to 11d using the constant current pulse width modulation means and the individual light quantity control means based on the supplied various dimming signals.

Next, the specific configuration of the dimming circuit 9 in the CCU 3 shown in Fig. 1 will be explained below with reference to Figs. 5 to 8.

As shown in Fig. 5, the dimming circuit 9 which constitutes the dimming control section includes an area dimming section 17 to which the video signal from the video signal processing circuit 8 is inputted via the input terminal 3a, and a full-screen brightness control section 18 to which the various dimming signals generated by the area dimming section 17 are supplied.

The area dimming section 17 divides an observation image based on the inputted video signal, that is, the screen 16 of the monitor 15 shown in Fig. 4 into 4 screen illumination area, the same number of the 4 LEDs 11a to 11d, and also detects the brightness of the screens 16a to 16d using the inputted video signal and based on the luminance signals of the screens 16a to 16d that correspond to each of the illumination areas for the divided screens 16a to 16d. And the area dimming section 17 compensates each light quantity based on the detected result so as to generate dimming signals for the areas 1 to 4, which are supplied to the full-screen brightness control section 18.

Specifically, the area dimming section 17 is provided with, as shown in Fig. 6, area 1 to 4 masking sections 19a to 19d corresponding to each of the LEDs 11a to 11d to which video signals are supplied via an input terminal 17a, and first to fourth dimming circuit sections 20a to 20d to which the outputted signals from the area 1 to 4 masking sections 19a to 19d.

The area 1 to 4 masking sections 19a to 19d detect the brightness of the screens 16a to 16d using the inputted video signals and based on the luminance signals of each of the screens 16a to 16d corresponding to the illumination areas of each of the LEDs 11a to 11d, and supplies the detected results to the first to fourth dimming circuit sections 20a to 20d in the following stage.

In the case, the area 1 masking section 19a detects the brightness using the inputted video signals and based on the luminance signals of the screen 16a shown in Fig. 4 corresponding to the illumination area of the LED 11a. The area 2 masking section 19b detects the brightness using the inputted video signals and based on the luminance signals of the screen 16b shown in Fig. 4 corresponding to the illumination area of the LED 11b. The area 3 masking section 19c detects the brightness using the inputted video signals and based on the luminance signals of the screen 16c shown in Fig. 4 corresponding to the illumination area of the LED 11c. The area 4 masking section 19d detects the brightness using the inputted video signals and based on the luminance signals of the screen 16d shown in Fig. 4 corresponding to the illumination area of the LED 11d.

The first to fourth dimming circuit sections 20a to 20d compare each of the supplied detected results with a predetermined reference value (which is a threshold and changeable), and adequately compensates the light quantities, so as to generate the dimming signals for the areas 1 to 4 corresponding to each of the LEDs 11a to 11d. Then, the first to fourth dimming circuit sections 20a to 20d supply the dimming signals for the areas 1 to 4 to the full-screen brightness control section 18 shown in Fig. 5 via the output terminals 21 a to 21d respectively.

As a result, the area dimming section 17 shown in Fig. 5 detects the brightness of each of the illumination area of the LEDs 11a to 11d, and each of the detected results is compared with a reference value, so that the dimming signals for the areas 1 to 4 are obtained after the compensation for a proper light quantity.

Therefore, the driving of each of the LEDs 11a to 11d based on the obtained dimming signals for the areas 1 to 4 provides a proper dimming even when a part of the entire field of vision for observation by the endoscope 2 having brighter parts and darker parts locally provides an observation image of the endoscope.

Even when each of the LEDs 11a to 11d is properly dimmed as described above, the entire screen 16 of the monitor 15 desirably has a brightness to be easily observed.

Thus, in the present embodiment, when each of the LEDs 11a to 11d is optimally dimmed as described above, the brightness of the entire screen 16 of the monitor 15 can be controlled by the full-screen brightness control section 18 shown in Figs. 7 and 8 so that the entire screen 16 has a brightness for easier observation, for example, by increasing the brightness when it is too dark and decreasing the brightness when it is too bright.

That is, the full-screen brightness control section 18 which constitutes the dimming control section is able to control the dimming so that the entire screen 16 for an observation image has a brightness at a constant value.

Specifically, the full-screen brightness control section 18 includes: as shown in Fig. 7, input terminals 22a to 22d to which dimming signals for areas 1 to 4 are supplied from the area dimming section 17 shown in Fig. 6; an adder 23 for adding the dimming signals for areas 1 to 4 from the input terminals 22a to 22d respectively; a coefficient computing section 24 for computing coefficients by inputting the outputted signals from the adder 23 and outputting the coefficients; multipliers 25a to 25d for amplifying the signal levels of the dimming signals for areas 1 to 4 from the input terminals 22a to 22d by multiplying the coefficients from the coefficient computing section 24; and output terminals 26a to 26d for outputting the outputted signals from the multipliers 25a to 25d to the downstream LED drive circuit 10.

The specific configuration example of the full-screen brightness control section 18 is shown in Fig. 8.

As shown in Fig. 8, the full-screen brightness control section 18 includes: first to third adders 23a to 23c as the adder 23; a comparator 24A as the coefficient computing section 24; a reference value generating section 25 for generating and outputting a reference value such as a reference time to the comparator 24A at one input terminal thereof; and the multipliers 25a to 25d for amplifying the signal levels based on the comparison result from the comparator 24A.

To the other input terminal of the comparator 24A, the adding result added by the first to third adders 23a to 23c is supplied. Then, the comparator 24A compares the adding result with a reference value such as a reference time, so as to supply the comparison result to the multipliers 25a to 25d as a coefficient. For example in the case where the adding result is twice that of the reference time, the comparison result is 1/2, which is the coefficient.

In the configuration example shown in Fig. 8, the dimming signals for areas 1 to 4 which are the outputted signals from the multipliers 25a to 25d respectively are supplied to the LED 1 to 4 drive circuits 10a to 10d that correspond to each of the LEDs 11a to 11d configuring the LED drive circuit 10.

Then, each of the LED 1 to 4 drive circuits 10a to 10d obtains the dimming signals for areas 1 to 4 which are compensated for proper light quantities based on the supplied dimming signals for areas 1 to 4 respectively. Therefore, the driving of each of the LEDs 11a to 11d based on the obtained dimming signals for the areas 1 to 4 provides a proper dimming even when a field of vision for observation by the endoscope 2 having brighter parts and darker parts locally provides an observation image of the endoscope.

An LED 1 dimming mask section 20A shown in Fig. 8 is configured with the an area 1 masking section 19a shown in Fig. 6, and the first dimming circuit section 20a. And so the LED 2 dimming mask section 20B to LED 4 dimming mask section 20D are configured with area 2 to 4 masking sections 19b to 19d shown in Fig. 6 that correspond to the LED 11b to 11d and the second to fourth dimming circuit sections 20b to 20d respectively, as in the case of the LED 1 dimming mask section 20A.

Here, a specific example of the control by the full-screen brightness control section 18 will be explained below with reference to Fig. 9A and Fig. 9B. For example, as shown in A to D of Fig. 9A, the dimming signals for areas 1 to 4 supplied to the full-screen brightness control section 18 are the ones that are compensated so as to have proper light quantity according to the illuminating areas of the each of the LEDs 11a to 11d (each of the screens 16a to 16d shown in Fig. 4) using the input video signals by the area dimming section 17 in the dimming circuit 9 of the present embodiment.

Usually, the dimming circuit 9 is able to control the light quantity of a corresponding LED by performing a process for controlling a pulse width P1 in one frame in various dimming signals, as shown in A to D of Fig. 9A, as a process for compensating light quantity.

The examples of dimming signals for areas 1 to 4 which are compensated to have a pulse width P1 by the area dimming section 17 as described above are shown in A to D of Fig. 9A. The E in Fig. 9B shows an integration time tL of the pulse width P1 of the dimming signals for areas 1 to 4 which is added by the adder 23 (the first to third adders 23a to 23c in Fig. 8), while the F in Fig. 9B shows a reference time which is a reference value used in the coefficient computing section 24 (a reference time which is the reference value from the reference value generating section 25 in Fig. 8) tO.

Then, the coefficient computing section 24 detects, for example, that the integration time of the pulse width P1 of each of the supplied dimming signals for areas 1 to 4 is equal to the integration time tL shown in E of Fig. 9. After that, the coefficient computing section 24 compares the integration time tL with the reference time tO and obtains a comparison result that integration time tL is generally twice that for the reference time tO. So, the coefficient computing section 24 sets a coefficient to be 1/2, which is supplied to each of the multipliers 25a to 25d.

That is, because the entire screen 16 of the monitor 15 has brightness which is twice that of the reference value, the coefficient is set to be 1/2, which is used to reduce each signal level to a half of that by multiplying each of the dimming signals for areas 1 to 4 by the coefficient 1/2 by each of the multipliers 25a to 25d.

This causes the integration time of each pulse width P1 of the outputted signal from the full-screen brightness control section 18 to be an integration time tO1 which is generally the same as the reference time tO, as shown in G of Fig. 9B, for example. That is, when each of the LEDs 11a to 11d is driven by the LED drive circuit 10 (the LED 1 to 4 drive circuits 10a to 10d) based on the dimming signals for areas 1 to 4 which are compensated as described above, the entire screen 16 of the monitor 15 can have brightness which is reduced to a half of that at the first dimming. That is, the driving of each of the LEDs 11a to 11d is controlled so that the screen 16 of the monitor 15 has brightness which is generally the same as that for the reference time tO, which allows the entire screen 16 of the monitor 15 to have brightness for easier observation.

The coefficient by the coefficient computing section 24 and the reference value by reference value generating section 25 may be set as needed, and may be set by a user from outside.

Next, the operation of the above configuration will be explained below with reference to Fig. 10 to Fig. 13.

In using the endoscope apparatus 1 of the present embodiment, the LED drive circuit 10 and the CCD driver 13 of the CCU 3 are driven. The LED drive circuit 10 of the CCU 3 causes the LEDs 11a to 11d of the endoscope 2 to be driven and the LEDs 11a to 11d are turned on, so that illumination lights are emitted from the image pickup unit 7 in the distal end portion 5 of the insertion portion 4 in the direction toward a field of vision for observation by the endoscope 2.

When the CCD driver 13 of the CCU 3 causes the CCD 12 of the endoscope 2 to be driven, an observation image of the endoscope 2 is outputted as a converted electrical signal by the CCD 12. The outputted signal from the CCD 12 is inputted to the video signal processing circuit 8 to be converted to a video signal, thereby the video signal outputted from the video signal processing circuit 8 is inputted to the monitor 15, and the observation image of the endoscope 2 is displayed on the screen 16 of the monitor 15.

At this point, in an observation using the endoscope 2, assume that a subject 30 is disposed near the distal end surface 5A in the direction of the field of vision for observation of the distal end portion 5 in the insertion portion 4, for example in front of the lower LEDs 11b and 11c at the distal end surface 5A of the distal end portion 5 as an obstacle that blocks the irradiations, as shown in Fig. 10.

In such a case, in the dimming in the prior art, light quantities of the illumination lights for the entire field of vision for observation by the endoscope 2 are only controlled simultaneously, resulting in that the LEDs 11b and 11c which are disposed at the lower portion of the distal end surface 5A near the subject 30 are dimmed to have too large quantities of light, and the LEDs 11a and 11d at the upper portion of the distal end surface 5A are simultaneously dimmed to have the same light quantities. As a result, as shown in Fig. 11, the observation image of the subject 30 displayed on the screen 16 of the monitor 15 is too bright, and too dark in other body cavity due to a too small quantity of light.

Therefore, in the present embodiment, as described above, the area dimming section 17 shown in Fig. 5 divides an observation image based on the inputted video signal, that is the screen 16 of the monitor 15 shown in Fig. 4 into four screen areas to be illuminated, the same number as the four LEDs 11a to 11d, and detects the brightness of each of the screens 16a to 16d using the inputted video signal and based on the luminance signals of the screens 16a to 16d corresponding to the illumination areas of the divided screens 16a to 16d respectively, and then compensates each light quantity based on the detected result to generate dimming signals for areas 1 to 4, which are supplied to the full-screen brightness control section 18.

Specifically, the area 1 to 4 masking section 19a to 19d of the area dimming section 17 (see Fig. 6) detects the brightness based on the luminance signals of the screens 16a to 16d corresponding to the illumination areas of the LEDs 11a to 11d respectively using the inputted video signals, and supplies the detection result to the first to fourth dimming circuit sections 20a to 20d in the following stage respectively. Then, the first to fourth dimming circuit sections 20a to 20d individually compares each of the supplied detection results with predetermined reference values, and adequately compensates the light quantities, so as to generate dimming signals for areas 1 to 4 that correspond to the LED 11a to 11d respectively. Then, the first to fourth dimming circuit sections 20a to 20d supply the signals to the full-screen brightness control section 18 shown in Fig. 5 via the output terminals 21 a to 21d respectively.

In this way, the brightness of each illumination area by each of the LEDs 11a to 11d is individually detected by the area dimming section 17 shown in Fig. 5, and each detection result is compared with a reference value, so as to obtain dimming signals for areas 1 to 4 after compensation for a proper light quantity.

Then, in the present embodiment, the screen brightness control section 18 shown in Fig. 5, Fig. 7, and Fig. 8 performs a compensation process to cause the entire screen 16 of the monitor 15 to have a brightness for easier observation.

In the case, in the full-screen brightness control section 18, the coefficient computing section 24 detects, for example, that the integration time of the pulse widths P1 of the dimming signals for areas 1 to 4 supplied from the adder 23 is equal to the integration time tL shown in E of Fig. 9B. Then, the coefficient computing section 24 obtains the comparison result that the integration time tL is generally twice that for the reference time tO as the comparison result between the integration time tL and the reference time tO. So, the coefficient computing section 24 sets the coefficient to be 1/2, which is supplied to each of the multipliers 25a to 25d.

That is, because the entire screen 16 of the monitor 5 has the brightness which is twice that of the reference value, the coefficient is set to be 1/2, which is used to reduce each signal level to a half of that by multiplying each of the dimming signals for areas 1 to 4 by the coefficient by each of the multipliers 25a to 25d.

This causes the integration time of each pulse width P1 of the outputted signal from the full-screen brightness control section 18 to be an integration time tO1 which is generally the same as the reference time tO, as shown in G of Fig. 9B, for example. That is, when each of the LEDs 11a to 11d is driven by the LED drive circuit 10 (the LED 1 to 4 drive circuits 10a to 10d) based on the dimming signals for areas 1 to 4 which are compensated as described above, the entire screen 16 of the monitor 15 is controlled to have brightness which is reduced to a half of that at the first dimming. That is, the driving of each of the LEDs 11a to 1d is controlled so that the screen 16 of the monitor 15 has brightness which is generally the same as that for the reference time tO, which allows the entire screen 16 of the monitor 15 to have brightness for easier observation.

For example, according to the present embodiment, as shown in Fig. 12, the LEDs 11b and 11c which are disposed at the lower portion of the distal end surface 5A near the subject 30 are dimmed to have a light quantity for a darker image than that in the prior art shown in Fig. 10, and at the same time the other LEDs 11a and 11d which are disposed at the upper portion of the distal end surface 5A are dimmed to have a light quantity for a brighter image than that in the prior art. Then, the full-screen brightness control section 18 performs a compensation process to cause the entire screen 16 of the monitor 15 to have a brightness for easier observation.

As a result, the observation image displayed on the screen 16 of the monitor 15 has, as shown in Fig. 13, brightness that allows the subject 30 to be easily observed and facilitates the identification, and brightness for easier observation of the body cavity other than the subject 30 due to a larger quantity of light.

Therefore, according to the embodiment 1, even when an entire field of vision for observation by the endoscope 2 having brighter parts and darker parts locally provides an observation image of the endoscope, each of the LEDs 11a to 11d is adequately dimmed, and also the entire screen 16 of the monitor 15 is dimmed to have brightness for an easier observation.

In addition, each of the LEDs 11a to 11d of the image pickup unit 7 is individually controlled according to the brightness of the illumination area of each LED via the dimming circuit 9, thereby each of the divided screens 16a to 16d of an observation image by the endoscope 2 always has the most adequate light quantity.

In the embodiment 1, the light quantity of each LEDs 11a to 11d of the image pickup unit 7 may be controlled by controlling the current supplied to each of the LEDs 11a to 11d.

### (Embodiment 2)

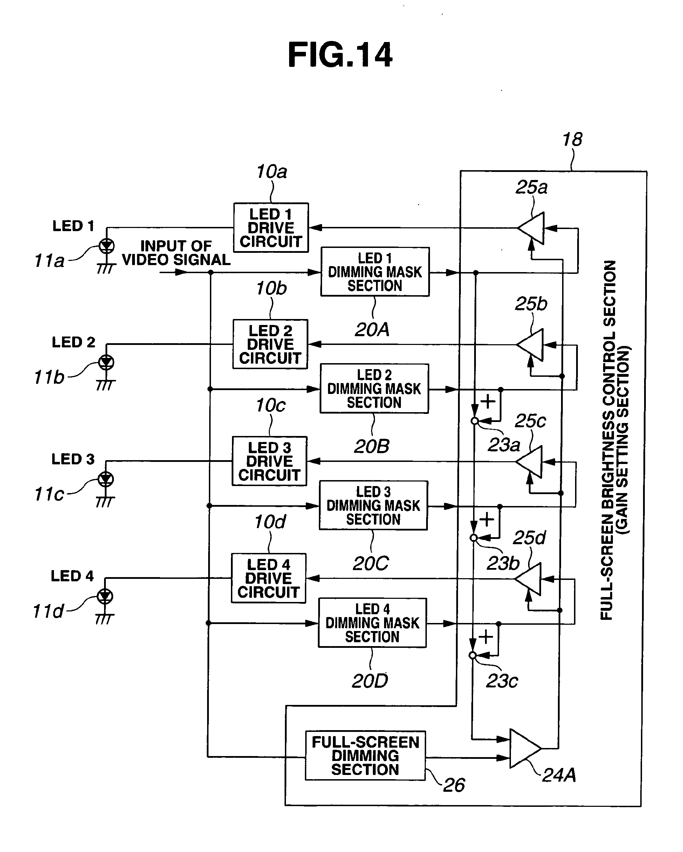

Fig. 14 is a configuration diagram showing an electrical circuit configuration of an entire endoscope apparatus according to an embodiment 2 of the present invention.

The endoscope apparatus 1 in the embodiment 2, as shown in Fig. 14, the full-screen brightness control section 18 is configured with a full-screen dimming section 26 instead of the reference value generating section 25 used in the embodiment 1.

The full-screen dimming section 26 generates a coefficient that causes the entire screen 16 of the monitor 15 shown in Fig. 4 to have a uniform brightness based on the inputted video signal, and outputs the coefficient to one input terminal of the comparator 24A that constitutes the coefficient computing section 24. That is, the full-screen dimming section 26 generates a coefficient that causes the entire screen including the divided screens 16a to 16d of the screen 16 shown in Fig. 4 to constantly have a uniform brightness, and outputs the coefficient.

By electrically connecting the full-screen dimming section 26 to the CCU 3 or an operation section (not shown) provided in the hand-side end portion 6 and by manipulating the operation section, the coefficient generated by the full-screen dimming section 26 may be automatically changed and set in accordance with a plurality of dimming modes. In the case, the full-screen dimming section 26 may include a memory section therein for having stored coefficients which are set in accordance with the dimming modes in advance.

Therefore, in the full-screen brightness control section 18 having such a configuration, the coefficient from full-screen dimming section 26 is supplied to one input terminal of the comparator 24A as the coefficient computing section 24, as in the case of the embodiment 1. Then, the comparator 24A compares, for example, the integration time of each pulse width P1 of the supplied dimming signals for each of areas 1 to 4 with the reference time which is the coefficient, and supplies the coefficient which is 1/2 when the integration time tL is generally twice that for the reference time tO as in the case of the embodiment 1 to each of the multipliers 25a to 25d.

Then, as in the case of the embodiment 1, the coefficient which is set to be 1/2 is used to reduce each signal level to a half of that by multiplying each of the dimming signals for areas 1 to 4 by the coefficient by each of the multipliers 25a to 25d. Based on the dimming signals for areas 1 to 4 after the compensation process as described above, each of the LED 11a to 11d is driven by the LED drive circuit 10 (the LED 1 to 4 drive circuits 10a to 10d). That is, the driving of each of the LED 1 1a to 11d is controlled to cause the screen 16 of the monitor 15 to have brightness which is in accordance with the predetermined coefficient, so that the entire screen 16 of the monitor 15 has brightness for easier observation.

Therefore, according to the embodiment 2, the same effect as that in the embodiment 1 can be obtained.

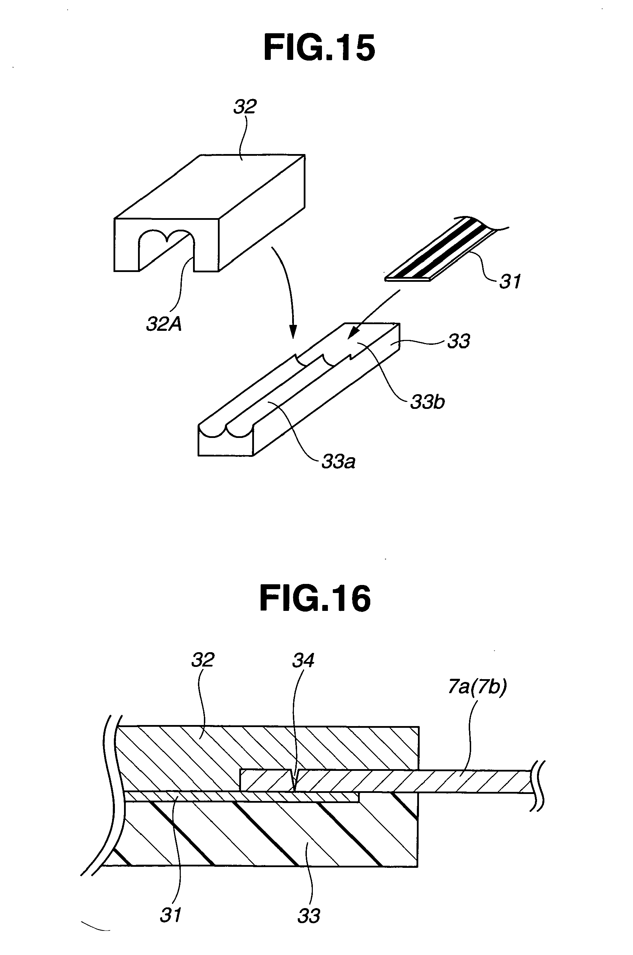

The endoscope apparatus 1 of the present invention is devised to reduce the manufacturing cost in consideration of the case where the insertion portion 4 of the endoscope 2 is designed to be disposable. The technology applied to such endoscope apparatus 1 according to the present invention will be explained below with reference to Figs. 15 to 17.

Figs. 15 and 16 illustrate the configuration of the connection A between the image pickup unit and a connecting line in the distal end portion of the endoscope. Fig. 15 is an exploded perspective diagram showing the configuration of a connection mechanism used at the connection A. Fig. 16 is a cross sectional diagram showing a fixed connection mechanism;

Generally, in many cases, the electrical connection of the distal end portion 5 of the endoscope 2 to the image pickup unit 7 is made with a flexible board in terms of compactness and the freedom of the design.

So, the endoscope apparatus 1 according to the present invention uses an top cover 32 and a cable receiver 33 as shown in Fig. 15 which constitutes a connection mechanism, so as to electrically secure a flexible board 31 extending from the image pickup unit 7 with the connecting line 7a (7b) which is the cable arranged in the insertion portion 4.

The top cover 32 accommodates, as shown in Fig. 15, each of the connecting line 7a (7b) in the inner periphery thereof, and also has a receiving groove 32A formed therein to be fitted with the cable receiver 33. The receiving groove 32A is provided with a through blade 34, on the distal end side thereof as shown in Fig. 16, which is protruded in the direction toward the cable receiver 33 and has a sharply tapered distal end portion formed of a conductive member.

The top cover 32 and the through blade 34 may be integrally formed of a conductive member, or only the through blade 34 may be configured as a conductive member.

The cable receiver 33 is configured to have a first receiver 33a for holding the connecting line 7a thereon and a second receiver 33b for holding the flexible board 31 which is extended from the image pickup unit 7 thereon.

When the flexible board 31 from the image pickup unit 7 is electrically connected to the connecting line 7a (7b) for securing, an operator places the flexible board 31 on the second receiver 33b of the cable receiver 33, and then places the connecting line 7a (7b) on the first receiver 33a of the cable receiver 33. In the placement, the connecting line 7a (7b) is placed with the distal end portion thereof being superimposed on the distal end portion of the flexible board 31 as shown in Fig. 16.

After that, the operator puts the top cover 32 on the cable receiver 33 in the above state. In the putting, the cable receiver 33 is ensured to be accommodated in the receiving groove 32A of the top cover 32, and the top cover 32 is pressed down so that the top cover 32 is fitted with the cable receiver 33.

Then, the through blade 34 of the top cover 32 abutts on or pierces into the flexible board 31 through the connecting line 7a (7b), the flexible board 31 being disposed under the connecting line 7a (7b). That is, the through blade 34 formed of a conductive member makes the connecting line 7a (7b) and the flexible board 31 electrically connected to each other, and at the same time maintains the secured state. The secured state is further ensured by the fitting between the top cover 31 and the cable receiver 33.

Therefore, such a connection mechanism facilitates the ensured electrical connection between the flexible board 31 from the image pickup unit 7 in the distal end portion 5 and the connecting line 7a (7b), and also maintains the electrically connected state and makes the elements fixedly secured to each other.

This provides an advantage that considerably contributes to the reduction of manufacturing cost. In addition, the connection mechanism can have a down-sized configuration, which saves space, and allows the connection mechanism to be mounted in the distal end portion 5, resulting in that it can considerably contribute to the down-sizing of the distal end portion 5 and thinning of the insertion portion 4.

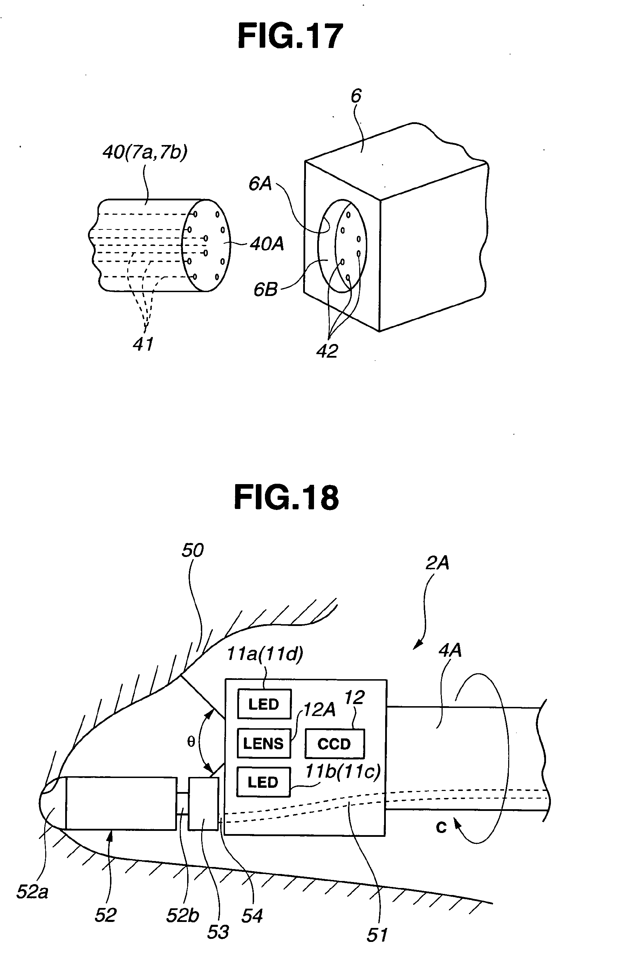

Fig. 17 is a perspective diagram illustrating the configuration of a connection mechanism used at the connection B between a proximal end portion of the insertion portion and the hand-side end portion of the endoscope.

When the insertion portion 4 of the endoscope 2 is configured to be disposable, the insertion portion 4 should be removably configured to the hand-side end portion 6 which constitutes an operation section.

Thus, in the endoscope apparatus 1 of the present invention, as shown in Fig. 16, a cable proximal end portion 40 and a cable receiver 6A which constitute a connection mechanism is used to electrically connect and secures the connecting lines 7a and 7b from the proximal end side of the insertion portion 4 to a connecting line (not shown) disposed on the hand-side end portion 6 side.

Specifically, as shown in Fig. 16, on the proximal end side of the insertion portion 4, the cable proximal end portion 40 is provided as a connector configuring a connection mechanism. The cable proximal end portion 40 is, for example, a cylindrical element formed of a material such as a resin with a connection cable 41 therein which includes the connecting lines 7a and 7b inserted through the insertion portion 4, for example.

In the case, a method for aligning the connection cable 41 in the cable proximal end portion 40 is not particularly limited. For example, through holes may be formed in the cable proximal end portion 40 in advance for inserting the connection cable 41 therethrough and after that, a resin is welded and thereby alignment of the connection cable 41 may be performed.

Moreover, the parts of a plurality of connection cables 41 which are exposed at the proximal end surface 40A of the cable proximal end portion 40 are individually provided with a plurality of metal terminals.

The cable receiver 6A as a connector receiver provided in the hand-side end portion 6 is formed in a shape corresponding to the shape of the cable proximal end portion 40. That is, the cable receiver 6A is formed as a cylindrical receiving groove which is recessed so that the cable proximal end portion 40 can be fitted therein.

The cable receiver 6A has a contact surface 6B provided with a plurality of electrical terminals 42 which contact and are electrically connected to the electrical terminals at the proximal end surface 40A of the cable proximal end portion 40.

In order to ensure the alignment between each electrical terminal at the cable proximal end portion 40 and each electrical terminal at the cable receiver 6A, for example, the proximal end surface 40A of the cable proximal end portion 40 may be configured to have a projection at a part of the outer periphery thereof, and the cable receiver 6A may be configured to have a engaging groove which is engaged with the projection at a part of the inner periphery thereof, so that the various electrical terminals can be aligned. Of course, the other methods may be used.

When the cable proximal end portion 40 of the insertion portion 4 and the cable receiver 6A of the hand-side end portion 6 are electrically connected and secured to each other, in the case where aligning means (not shown) such as the above described projection is provided, first, a operator fits the cable proximal end portion 40 into the cable receiver 6A so that the projection is engaged into the locking groove.

In the case, the operator presses the cable proximal end portion 40 into the cable receiver 6A so that the proximal end surface 40A contacts the contact surface 6B.

Thus, the contact between the proximal end surface 40A of the cable proximal end portion 40 and the contact surface 6B of the cable receiver 6A results in the contact between each of the electrical terminals at the cable proximal end portion 40 for electrical conduction, and at the same time the fixed secure connection being held under the electrical conduction, because the cable proximal end portion 40 is ensured to be fitted in the receiving groove of the cable receiver 6A.

Therefore, the insertion portion 4 should be configured to be removably attached to the hand-side end portion 6 when the insertion portion 4 is designed as a disposable element, but such a connection mechanism facilitates the ensured electrical connection between the insertion portion 4 and the hand-side end portion 6 with a simple configuration without using an expensive member such as a connector. This provides an advantage that considerably contributes to the reduction of manufacturing costs.

The endoscope apparatus 1 of the present invention is furthermore devised to enhance the insertability and the observability.

The technology applied such endoscope apparatus 1 according to the present invention will be explained below with reference to Fig. 18.

Fig. 18 is a cross sectional diagram showing a distal end portion of the insertion portion which constitutes a rotary self-propelled endoscope and has a leading portion in front of the distal end portion thereof.

In an endoscope apparatus of the prior art, a smooth movement of the endoscope insertion portion to a target site in a body cavity requires specialized skills. In addition, the general method for inserting the insertion portion utilizes the enlargement of the conduit and the linearization of large intestine by air supply, which may cause pain in a patient when the air is supplied or the intestine is intentionally deformed.

Thus, the endoscope apparatus of the present invention is improved so that the insertion portion becomes automatically insertable and can be smoothly inserted without the need of the conventional manipulation procedure for insertion and without deforming the intestine by using the elasticity of a leading portion that is provided at the distal end portion of the endoscope.

Specifically, the endoscope 2A is, for example, configured as a rotary self-propelled endoscope. That is, as shown in Fig. 18, the insertion portion 4A of the endoscope 2A is configured to be disposable, and includes an insertion portion body (not shown) and a rotary barrel 4A.

Not shown, but the insertion portion body has various tubes for an inner tube, connecting lines 7a and 7b, conduits for air supply and water supply, a conduit for treatment instrument, and the like disposed therein.

The rotary barrel 4A is configured to be rotatable about its axis (for example, in the direction of the arrow C in Fig. 18), and is a flexible barrel formed by winging a metal plate member (not shown) which is machined to have a convex-concave profile in cross section and has biocompatibility. In the rotary barrel 4A, the convex-concave profiles are engaged with each other generally without gaps therebetween, and a helix-shaped portion (not shown) which forms a helical convex portion (or helical concave portion, or convex portions which are provided in a protruded manner to be connected along the helix) is formed on the outer peripheral surface.

When the rotary barrel 4A turns in circular motion, the helix-shaped portion (not shown) on the outer peripheral surface contacts the inner wall of a body cavity of a subject, which generates a thrust that causes the rotary barrel 5A to move in the direction of insertion by itself.

In the situation, the thrust is applied so that the entire insertion portion 4 including the distal end portion 5 is caused to be advanced into a deeper part of the body cavity. The rotating drive force of the rotary barrel 4A is applied by a motor (not shown) which is mounted in the hand-side end portion 6 for example.

In such a rotary self-propelled endoscope 2A, an opening for air supply and water supply (not shown) of the distal end surface 5A of the distal end portion 5 is provided with a leading portion 52 mounted thereto as an insertion support. The leading portion 52 is mounted via a first connection 54, a base member 53, and a second connection 52b which are provided at an air supply conduit opening (not shown) at the distal end surface 5A.

The leading portion 52 is mounted so that the first connection 54 closes the air supply conduit opening (not shown), but the configuration is not limited to the above example, and the leading portion 52 may be formed in a hood shape having a distal end portion 52a for covering the entire distal end portion 5 for example.

In addition, the distal end portion 52a of the leading portion 52 is formed in an arc shape or a tapered shape that does not scratch the wall of intestine. The entire leading portion 52 may have a shape which tapers from the proximal end side to the distal end portion 52a so that the leading portion 52 does not affect the viewing angle θ of the observation window 14. However, the shapes of the leading portion 52 and the distal end portion 52a are not limited to the above examples, and may be any shape that does not scratch the wall of intestine or affect the viewing angle θ of the observation window 14.

The second connection 52d is formed with an elastic member. Thus, when the insertion portion 4 is inserted into a body cavity, the leading portion 52 connected to the second connection 52d can be deformed upwardly, downwardly, rightwardly, and leftwardly due to the elasticity of the second connection 52d, and expand the wall of intestine without hurting.

The first connection 54 can be easily separated from the distal end portion 5 by air pressure through the air supply conduit (conduit for treatment instrument) 51 which is provided in the distal end portion 5 and the insertion portion 4, when the distal end portion 5 reaches a target site such as caecum for example. That is, when the observation or treatment of a target site is completed, the first connection 54 is used to separate the leading portion 52.

The separation of the leading portion 52 via the first connection may be performed by inserting a treatment instrument through the air supply conduit (conduit for treatment instrument) 51 and using the inserted treatment instrument.

The other configurations are similar to those in the embodiment 1 and the embodiment 2. Also, a lens 12A which constitutes an objective optical system is provided in front of the CCD 12 of the distal end portion 5, and in front of the lens 12A, the observation window 14 (see Fig. 3) is provided as in the case of the embodiment 1.

The range of the field of vision of the lens 12A and the observation window 14 of the endoscope 2A is the viewing angle θ as shown in Fig. 18, and observations and treatments can be performed with the viewing angle θ which is not influenced for the observations even when the leading portion 52 is provided.

Next, the operation of such an endoscope 2A will be explained below. The following will be described in the context of an examination of large intestine by endoscope.

Now, assume that an examination of large intestine is performed using the endoscope apparatus 1 for example. First, a surgeon inserts the insertion support (not shown) into the anus of a patient who is lying on a bed for example. Then, the surgeon moves the insertion portion 4 shown in Fig. 18 from the anus into intestinum rectum via the insertion support, and then rotates the rotary barrel (helix-shaped portion) 4A of the insertion portion 4 about the longitudinal axis thereof by operating a foot switch (not shown) by foot or operating an advancing/retracting switch (not shown) mounted to the hand-side end portion 6 or the CCU 3 by hand while grasping the grasping portion of the hand-side end portion 6.

Then, a rotating force is transmitted in the rotary barrel 4A from the proximal end portion to the distal end side thereof, as the result of that the entire rotary barrel 4A is rotated in a predetermined direction about its axis as shown by the arrow C of Fig. 18, which causes a thrust. The thrust of the rotary barrel 4A causes the insertion portion 4 to be advanced into a deeper portion of the large intestine.

All what the surgeon has to do is to softly hold the insertion support (not shown) and does not have to grasp and advance the insertion portion 4, so that only the thrust of the rotary barrel 4A allows the insertion portion 4 to be advanced into a deeper portion of the large intestine.

At this point, the helix-shaped portion (not shown) of the rotary barrel 4A contacts the ligaments of intestine wall in a relationship of a male screw and a female screw. The helix-shaped portion contacts the ligaments of intestine wall, which generates a thrust or the like that causes the helix-shaped portion to be smoothly advanced, resulting in the advancement of the insertion portion 4A from rectum to sigmoid flexure.

Then, using the bending operation of the bending portion (not shown) of the insertion portion 4, the insertion portion 4 smoothly passes through the sigmoid flexure, and then is smoothly advanced along the walls of the flexure between sigmoid flexure and non-movable descending colon, splenic flexure between the descending colon, and movable transverse colon, and hepatic flexure between the transverse colon and ascending colon, so as to reach the position near caecum which is a target site for example without change the functional state of the large intestine.

In the case, the leading portion 52 connected to the second connection 52d can be deformed upwardly, downwardly, rightwardly, and leftwardly due to the elasticity of the second connection 52d, and expand the wall of intestine without hurting, so that the distal end portion 5 of the insertion portion 4 can be smoothly advanced by keeping a sufficient range of the field of vision.

When the distal end portion 5 of the insertion portion 4 reaches the position near caecum, the surgeon supplies air through the air supply conduit (conduit for treatment instrument) 51 which is provided in the distal end portion 5 of the insertion portion 4, so that the air pressure causes the first connection 54 to be removed from the air supply conduit opening (not shown) to separate the leading portion 52 with the base member 53.

This provides the air supply conduit opening (not shown) in the distal end surface 5A of the distal end portion 5 for air supply and the like, and also provides a sufficient viewing angle θ, resulting in a good observation image of the large intestine.

After that, the surgeon rotates the rotary barrel 4A in the opposite way to that for the insertion, so as to move the insertion portion 4 backward in the direction for expelling the distal end portion 5 from the position near caecum at the deep part of the large intestine for the examination of large intestine. In the case also, the surgeon can move insertion portion 4 backward using the backward thrust of the rotary barrel 4A, without touching the insertion portion 4.

Therefore, according to the endoscope 2A having the above described configuration, the insertion portion 4 of the endoscope 2A becomes automatically insertable and can be smoothly inserted without the need of the conventional manipulation for insertion and without considerably deforming the intestine by using the elasticity of a leading portion 52 that is provided at the distal end portion 5 of the endoscope 2A. When the insertion portion 4 is expelled after the leading portion 52 is separated from the distal end portion 5, a sufficient viewing angle θ is provided, thereby an observation can be performed without losing any capability for observation.

The present invention described in the above embodiments is not limited to the embodiments, and various modifications can be implemented in the practical phase without departing from the gist of the present invention. Furthermore, the embodiments includes inventions at various stages, and the plurality of disclosed elements may be conveniently combined and extracted as various inventions.

For example, if the problem described in the paragraphs of Means for Solving the Problem can be solved and the advantages described in the paragraphs of Advantage of the Invention can be obtained even when some elements of those disclosed in the above embodiments are eliminated, the configuration without the eliminated elements may be extracted as an invention.

This application claims the benefit of Japanese Patent Application No. 2006-228191 filed on August 24, 2006 in Japan, the disclosure of which is incorporated in the description, claims, and drawings of this application by reference.

## Claims

1. An endoscope apparatus, comprising:
image pickup means;
a plurality of illumination means for illuminating a field of vision the image of which is picked up by the image pickup means;
a dimming control section for detecting the brightness of the area illuminated by each of the plurality of illumination means using the video signals obtained from the image pickup means, and dimming the light of each of the illumination means based on the detected result,
with the plurality of illumination means being arranged at the positions corresponding to the areas illuminated by each of the illumination means with respect to the image pickup means when the dimming control section detects the brightness of the areas illuminated by each of the plurality of illumination means.

2. The endoscope apparatus according to claim 1, wherein
the plurality of illumination means are provided to be arranged centering the image pickup means near the image pickup means.

3. The endoscope apparatus according to claim 1, wherein
the dimming control section controls the dimming so that the entire screen for an observation image based on the video signal which is obtained after dimming the plurality of illumination means has a brightness at a constant value.

4. The endoscope apparatus according to claim 2, wherein
the dimming control section controls the dimming so that the entire screen for an observation image based on the video signal which is obtained after dimming the plurality of illumination means has a brightness at a constant value.

5. The endoscope apparatus according to claim 1, wherein
the image pickup means is a solid state image pickup device, and
the illumination means is an LED.

6. The endoscope apparatus according to claim 2, wherein
the image pickup means is a solid state image pickup device, and
the illumination means is an LED.

7. The endoscope apparatus according to claim 3, wherein
the image pickup means is a solid state image pickup device, and
the illumination means is an LED.

8. The endoscope apparatus according to claim 4, wherein
the image pickup means is a solid state image pickup device, and
the illumination means is an LED.

9. The endoscope apparatus according to claim 1, wherein
the image pickup means is provided at the distal end portion of the insertion portion of the endoscope, and
the plurality of illumination means is individually arranged centering the image pickup means near the image pickup means at the distal end surface of the distal end portion.

10. The endoscope apparatus according to claim 2, wherein
the image pickup means is provided at the distal end portion of the insertion portion of the endoscope, and
the plurality of illumination means is individually arranged centering the image pickup means near the image pickup means at the distal end surface of the distal end portion.

11. The endoscope apparatus according to claim 3, wherein
the image pickup means is provided at the distal end portion of the insertion portion of the endoscope, and
the plurality of illumination means is individually arranged centering the image pickup means near the image pickup means at the distal end surface of the distal end portion.

12. The endoscope apparatus according to claim 4, wherein
the image pickup means is provided at the distal end portion of the insertion portion of the endoscope, and
the plurality of illumination means is individually arranged centering the image pickup means near the image pickup means at the distal end surface of the distal end portion.

13. The endoscope apparatus according to claim 5, wherein
the image pickup means is provided at the distal end portion of the insertion portion of the endoscope, and
the plurality of illumination means is individually arranged centering the image pickup means near the image pickup means at the distal end surface of the distal end portion.

14. The endoscope apparatus according to claim 6, wherein
the image pickup means is provided at the distal end portion of the insertion portion of the endoscope, and
the plurality of illumination means is individually arranged centering the image pickup means near the image pickup means at the distal end surface of the distal end portion.

15. The endoscope apparatus according to claim 7, wherein
the image pickup means is provided at the distal end portion of the insertion portion of the endoscope, and
the plurality of illumination means is individually arranged centering the image pickup means near the image pickup means at the distal end surface of the distal end portion.

16. The endoscope apparatus according to claim 8, wherein
the image pickup means is provided at the distal end portion of the insertion portion of the endoscope, and
the plurality of illumination means is individually arranged centering the image pickup means near the image pickup means at the distal end surface of the distal end portion.
